# EUROPEAN PATENT APPLICATION

(11) **EP 4 616 903 A1**
(43) Date of publication of application: **17.09.2025**
(21) Application number: 23889142.8
(22) Date of filing: 08.11.2023
(51) Int. Cl.: A61N 7/02, A61N 7/00

(54) **THERAPEUTIC ULTRASOUND GENERATION DEVICE, AND HANDPIECE FOR ULTRASOUND TREATMENT INCLUDING SAME**

(30) Priority: 11.11.2022 KR 20220150239
(71) Applicant: CLASSYS INC., Seoul 06220 (KR)
(72) Inventor: PARK, Su Man, Yongin-si Gyeonggi-do 16821 (KR); PARK, Si Hyung, Seoul 08329 (KR)
(74) Representative: Wörz, Volker Alfred
(86) International application number: PCT/KR2023/017863
(87) International publication number: WO 2024/101884

(57) **Abstract**

The present invention relates to a therapeutic ultrasound generation device, and a handpiece for ultrasound treatment including same. By connecting an ultrasonic transducer unit with a rotating motor unit through an eccentric shat unit and rotating the ultrasonic transducer unit at an eccentric position on a plane to move the focus of the ultrasound waves in a circular manner on the plane, the invention can easly control the output of ultrasound waves to further improve the treatment effect, expands the range of movement radius of the focus to increase the versatility of treatment, and simplifies the structure which moves the focus of ultrasound eaves generated from the ultrasonic transducer unit in a circular manner on a plane, thereby minimizing the load generated on the motor and reducing manufacturing costs.

## Description

### [Technical Field]

The present disclosure relates to a therapeutic ultrasound generation device, and a handpiece for ultrasound treatment including same, and in more detail, to a therapeutic ultrasound generation device that can reduce the size of a handpiece for ultrasound treatment by simplifying a structure that moves the focus of ultrasonic waves generated by an ultrasound generation unit in a circular path on the same plane, and a handpiece for ultrasound treatment including the therapeutic ultrasound generation device.

### [Background Art]

In recent years, as dietary habits have become more Westernized, obesity has rapidly increased and has become the main factor that harms public health and beauty, and various diet programs and ultrasound obesity treatment devices have been developed and are widely used to treat it.

High-Intensity Focused Ultrasound (HIFU) technology for obesity treatment was originally used for the purpose of anticancer treatment to destroy cancer cells by selectively coagulating internal organ tumors at high temperatures in a non-invasive manner. However, Solta Medical in the United States was the first to develop a device called Liposonix, which is equipped with HIFU, for the purpose of treating abdominal obesity in the human body.

The process of fat destruction using HIFU (High-Intensity Focused Ultrasound) is that when focused ultrasonic waves are concentrated at a specific point in fat cells, the temperature of the tissue instantaneously rises to 65°C to 100°C, thereby causing tissue destruction.

Unlike other dermatological equipment such as laser and RF equipment, HIFU equipment induces coagulation necrosis of fat by non-invasively concentrating HIFU energy on a selected area without causing any damage to the skin surface. The necrotic fat cells are naturally removed by the body's repair mechanism for damaged parts.

An example of a known ultrasound obesity treatment device is Korean Patent No. 10-1365946 (published on February 24, 2014), titled "High Intensity Focused Ultrasound Generating Device for The Deduction of Fat Tissue".

The "High Intensity Focused Ultrasound Generating Device for The Deduction of Fat Tissue" penetrates ultrasonic waves into the skin by moving a transducer to a desired point in the X-axis and Y-axis directions and then pivoting it around the axes.

However, the "High Intensity Focused Ultrasound Generating Device for The Deduction of Fat Tissue" has a problem in that when ultrasonic waves are provided, they are delivered in an curved (arc) shape due to the characteristic of the pivot mechanism, so the energy delivered to the skin decreases and the focal depth changes toward the periphery, which results in uneven treatment.

In order to solve such a problem, the present applicant has proposed, in Korean Patent No. 10-1649899, titled "Ultrasonic Apparatus for Treatment", a structure that improves treatment performance by including a focal point rotating unit for circularly moving the focus of ultrasonic waves, which are generated from an ultrasound generation unit, on the same plane, forming the focus of the ultrasonic waves in a circular shape having a predetermined radius at a uniform depth in the skin, and uniformly applying energy within the radius.

However, Korean Patent No. 1649899, titled "Ultrasonic Apparatus for Treatment", has a structure in which a plurality of protruding members protruding at different heights are brought into contact with the top of the ultrasound generation unit, and thus has a limitation in reducing the size and also has a problem in that it is restricted in stably moving the focus of the ultrasonic waves, generated from an ultrasound transducer unit, in a circular path on the same plane.

Further, in Korean Patent No. 1649899, titled "Ultrasonic Apparatus for Treatment", since the ultrasound generation unit is rotated in a tilted state, ultrasonic waves are generated diagonally. As a result, the focal point of the ultrasonic waves is formed diagonally on the skin, so there is a problem in that it is difficult to uniformly transmit the intensity of the ultrasonic waves, i.e., the output of the ultrasonic waves, to the skin and it is difficult to increase the radius of the circle formed on the plane.

Further, Korean Patent No. 1649899, titled "Ultrasonic Apparatus for Treatment" has a structure in which a plurality of protruding members protruding at different heights is brought into contact with the top of the ultrasound generation unit and the ultrasound generation unit is rotated in a tilted state, so it has a problem in that the structure is complicated, the manufacturing cost increases, and a large load is required to rotate the ultrasound generation unit due to torsional moment.

### [Disclosure]

### [Technical Problem]

An objective of the present disclosure is to provide a therapeutic ultrasound generation device that improves obesity treatment performance by penetrating ultrasonic waves uniformly and evenly into the skin by moving the focus of the ultrasonic waves in a circular path at a uniform depth in the skin, and a handpiece for ultrasound treatment including the therapeutic ultrasound generation device.

Another objective of the present disclosure is to provide a therapeutic ultrasound generation device that makes it possible to easily adjust the output of ultrasonic waves by moving the focus of the ultrasonic waves in a circular path on the same plane by rotating an ultrasonic transducer unit at an offset position on a plane, that makes it possible to increase the radius of movement of the focus, and minimizes the load on a motor during operation, and a handpiece for ultrasound treatment including the therapeutic ultrasound generation device.

Another objective of the present disclosure is to provide a therapeutic ultrasound generation device that can reduce the size of a handpiece for ultrasound treatment by simplifying the structure for moving the focus of ultrasonic waves, generated from an ultrasonic transducer unit, in a circular path on the same plane, and enables ultrasound treatment on localized areas of a patient's skin, such as under the eyes, and a handpiece for ultrasound treatment including the therapeutic ultrasound generation device.

### [Technical Solution]

In order to achieve the objectives described above, an embodiment of a therapeutic ultrasound generation device according to the present disclosure includes: a cartridge housing having, on a bottom thereof, a window configured to come into contact with the skin, through which ultrasonic waves pass; an ultrasound transducer positioned to rotate on a plane in the cartridge housing and configured to generate ultrasonic waves; a rotating motor configured to rotate the ultrasound transducer; and an eccentric shaft eccentrically connected to a center of body of the ultrasound transducer on a top of the ultrasound transducer, and configured to rotate by rotational force transmitted from the rotating motor.

The ultrasound transducer may be rotated around the eccentric shaft, whereby a focus of ultrasonic waves generated by the ultrasound transducer may move in a circular path on a plane.

An embodiment of the therapeutic ultrasound generation device according to the present disclosure may further include: a power supply protruding terminal unit protruding upward from the ultrasound transducer and configured to supply power to the ultrasound transducer; and a power supply board disposed above and spaced apart from the ultrasonic transducer in the cartridge housing, to which the power supply protruding terminal unit is connected.

In the present disclosure, the cartridge housing may have a sealed internal structure and may be filled with an ultrasound transmission medium, and the ultrasound transmission medium may be an insulating liquid.

In the present disclosure, a ring-shaped electrode pattern connected through a power cable to receive power may be positioned on a bottom of the power supply board, and the power supply protruding terminal unit moves in a circle path along the ring-shaped electrode pattern in elastic contact with the ring-shaped electrode pattern.

In the present disclosure, the power supply protruding terminal unit may include: a jig pin member connected to the ring-shaped pattern and electrically connected to the ultrasound transducer through a wire; a pin insertion member positioned on the top of the ultrasonic transducer, in which the jig pin member is positioned to be movable up and down; and a pin support spring member positioned in the pin insertion member and configured to elastically support the jig pin member.

An embodiment of the therapeutic ultrasound generation device according to the present disclosure may further include: a board position-fixing board positioned between a top of the cartridge housing and the power supply board and configured to support the position of the power supply board; a plurality of first board fixing portions having both ends fixed to the top of the cartridge housing and the board position-fixing board and configured to support the position of the board position-fixing board; and a plurality of second board fixing portions having both ends fixed to the board position-fixing board and the power supply board and configured to support the position of the power supply board.

In the present disclosure, the plurality of first board fixing portions and the plurality of second board fixing portions may be formed in a hollow tubular shape, and power cables connecting the ring-shaped electrode pattern and a first main power supply terminal protruding upward from the cartridge housing may be disposed inside the first and second board fixing portions.

An embodiment of the therapeutic ultrasound generation device may further include an ultrasound transducer displacement unit under which the ultrasound transducer is movably positioned and to an upper portion of which the eccentric shaft is connected, thereby being able to adjust a movement radius of the focus on a plane.

In order to achieve the objectives described above, an embodiment of a therapeutic ultrasound generation device according to the present disclosure includes: a body housing connected to a control body through a handpiece cable assembly; and a therapeutic ultrasound generation device detachably coupled to the body housing, wherein the therapeutic ultrasound generation device includes an embodiment of the therapeutic ultrasound generation device according to the present disclosure.

### [Advantageous Effects]

The present disclosure applies energy uniformly and evenly to a treatment area by moving the focus of ultrasonic waves at a uniform depth within the skin in a planar manner, and applies energy uniformly and evenly within a radius by forming the focus of the ultrasonic waves at a uniform depth within the skin in a circular shape with a predetermined radius, thereby having an effect of improving treatment performance.

The present disclosure has the effect of further improving the treatment effect by moving the focus of ultrasonic waves along a circular path on a plane by rotating the ultrasonic transducer at an offset position on the plane to make it easy to adjust the output of ultrasonic waves, and has the effect of increasing the versatility of the treatment by expanding the range of the movement radius of the focus.

Further, the present disclosure has the effect of minimizing the load on the motor and reducing the manufacturing costs by simplifying the structure for moving the focus of the ultrasonic waves generated by the ultrasonic transducer along a circular path on the same plane.

### [Description of Drawings]

FIG. 1 is a perspective view showing an embodiment of a handpiece for ultrasound treatment according to the present disclosure.
FIG. 2 is an exploded perspective view showing an embodiment of a handpiece for ultrasound treatment according to the present disclosure.
FIG. 3 is a perspective view showing an embodiment of a therapeutic ultrasound generation device according to the present disclosure.
FIG. 4 is an enlarged cross-sectional view of the portion A of FIG. 3.
FIG. 5 is a partial enlarged bottom perspective view showing an embodiment of a therapeutic ultrasound generation device according to the present disclosure.
FIG. 6 is a partial enlarged view showing another embodiment of a therapeutic ultrasound generation device according to the present disclosure.

### * Description of Reference Numerals*

| | | | |
|---|---|---|---|
| 100 : | body housing | 101 : | cartridge lock |
| 110 : | handle | 111 : | handle bridge |
| 112 : | handle body | | |
| 200 : | cartridge housing | 201 : | upper housing |
| 201a : | cartridge board | 202 : | housing-sealing cap |
| 203 : | window | 210 : | medium supply protruding tube |
| 220 : | medium discharge protruding tube | 300 : | ultrasonic transducer |
| 400 : | rotating motor | 410 : | shaft adapter section |
| 500 : | eccentric shaft | 510 : | connection shaft section |
| 600 : | power supply protruding terminal unit | 600a : | cathode protruding terminal unit |
| 600b : | anode protruding terminal unit | 610 : | jig pin member |
| 620 : | pin insertion member | 630 : | pin support spring member |
| 700 : | power supply board | 710 : | ring-shaped electrode pattern |
| 711 : | first electrode pattern | 712 : | second electrode pattern |
| 800 : | board position-fixing board | 810 : | first board fixing portion |
| 820 : | second board fixing portion | 900 : | ultrasound transducer displacement unit |
| 1000 : | control body | | |

### [Best Mode]

Hereafter, the present disclosure is described in more detail.

Exemplary embodiments of the present disclosure are described hereafter in detail with reference to the accompanying drawings. Before describing the present disclosure, it should be noted that the terms or terminologies used herein and claims should not be construed as common meanings or the meanings in dictionaries. Therefore, the configurations described in the embodiments and drawings of the present disclosure are merely most preferable embodiments but do not represent all of the technical spirit of the present invention. Thus, it should be understood that the present disclosure should be construed as including all the changes, equivalents, and substitutions included in the spirit and scope of the present disclosure at the time of filing this application.

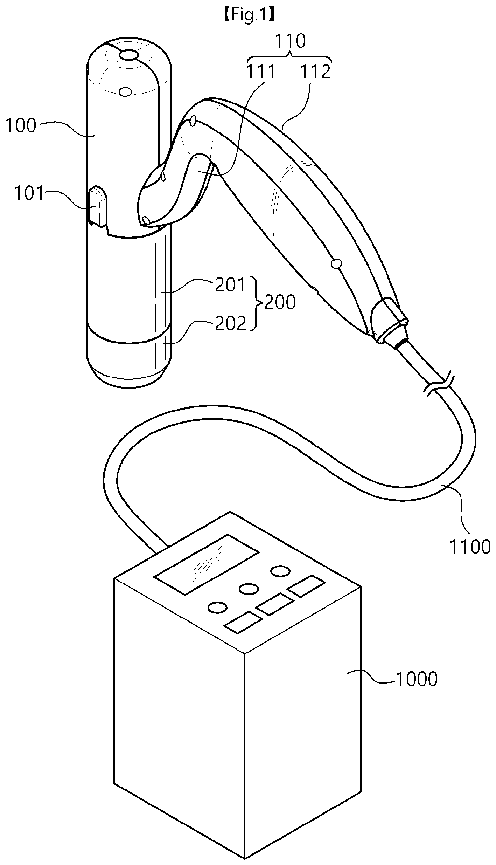

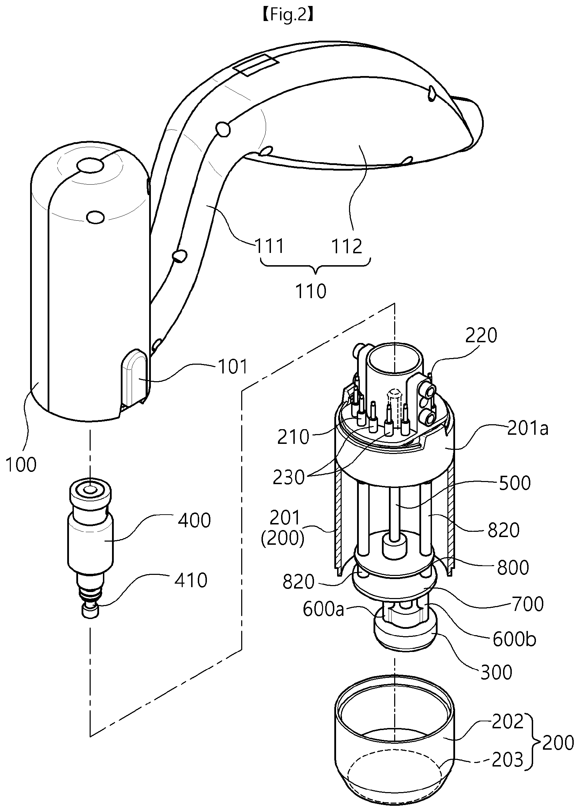

FIG. 1 is a perspective view showing an embodiment of a handpiece for ultrasound treatment according to the present disclosure and FIG. 2 is an exploded perspective view showing an embodiment of a handpiece for ultrasound treatment according to the present disclosure.

Referring to FIG. 1 and FIG. 2, a handpiece for ultrasound treatment according to the present disclosure includes a body housing 100 to which a therapeutic ultrasound generation device according to the present disclosure is detachably coupled.

An embodiment of the therapeutic ultrasound generation device according to the present disclosure includes a cartridge housing 200 in which an ultrasonic transducer 300 is positioned and which has a window 203 at its lower portion through which ultrasonic wave pass.

The therapeutic ultrasound generation device according to the present disclosure directs ultrasonic waves generated by the ultrasonic transducer 300 in the cartridge housing 200 to the skin by transmitting it through the window 203 positioned at the lower portion of the cartridge housing 200, and is detachably coupled to the body housing 100 of the handpiece for ultrasound treatment according to the present disclosure.

The handpiece for ultrasound treatment according to the present disclosure is connected to a control body 1000 through a handpiece cable assembly 1100, and the handpiece cable assembly 1100 includes a power cable, and a medium supply line and a medium discharge line for circulating a medium in the cartridge housing 200.

The handpiece cable assembly 1100 and the control body 1000 can be variously modified on the basis of known examples from ultrasound treatment devices well known in the art, and a more detailed description is omitted.

The ultrasonic transducer 300 has a preset number of uses, and the ultrasonic transducer 300 of the therapeutic ultrasound generation device according to the present disclosure is replaced with a new one when the preset number of uses of the ultrasonic transducer 300 is reached.

That is, the cartridge housing 200 is detachably coupled to the body housing 100, and the ultrasonic transducer unit 300 can be replaced after being used for the preset number of uses.

An embodiment of the therapeutic ultrasound generation device according to the present disclosure is described in detail hereafter.

A cartridge locking unit 101 that can hook and secure the cartridge housing 200 is positioned on the body housing 100.

The cartridge lock 101 is elastically supported by a spring, and a wedge-shaped locking portion is locked in a locking groove positioned inside the cartridge housing, thereby securing the coupled state of the cartridge housing 200. Further, when the cartridge lock 101 is pressed, the locking portion disengages from the locking groove, whereby the cartridge housing 200 can be separated.

In addition to the cartridge lock 101, a known detachable locking structure is applied to the cartridge housing 200, whereby the cartridge housing 200 can be kept coupled with the body housing 100 or separated, and so it should be noted that a more detailed description is omitted.

A handle 110, which an operator can hold by hand, is positioned on one side of the body housing 100.

The handle 110 includes a handle bridge 111 that bends upward from the body housing 100 and a handle body 112 that is curved downward from the handle bridge 111.

An operator can easily perform a procedure by holding the handle body 112 bending from the handle bridge 111 and integrally extending downward and easily bringing the window 203 of the cartridge housing 200 into close contact the skin.

The handle 110 has a shape bending upward and then extending downward and is designed such that the handle body 112 is held, so the load that is applied to an operator during a procedure can be minimized and it is possible to perform a procedure by easily bringing the window 203 of the cartridge housing 200 into close contact with the skin.

Meanwhile, the therapeutic ultrasound generation device according to the present disclosure includes the cartridge housing 200 having the window through which ultrasonic waves pass and which is positioned at the lower portion, and the ultrasonic transducer 300 positioned inside the cartridge housing 200 and generating ultrasonic waves downward.

The window 203 is made of a transparent or translucent material through which ultrasonic waves can pass, and is made of a known material that transmits ultrasonic waves, so it should be noted that a more detailed description is omitted.

The cartridge housing 200 includes an upper housing 201 that is detachably coupled to the body housing 100 and a housing-sealing cap 202 that is detachably coupled to the lower portion of the upper housing 201.

The window 203 is formed on the bottom of the housing-sealing cap 202 in a size that enables an ultrasound procedure in contact with the skin of a patient.

The cartridge housing 200 has a sealed internal structure and is filled with an ultrasound transmission medium.

It is exemplified that the ultrasound transmission medium is an insulating liquid, and the insulating liquid can be variously modified as well-known insulating liquids that is not electrically conductive, and thus, it should be noted that a more detailed description is omitted.

The ultrasound transmission medium not only serves to transmit ultrasonic waves but also serves to cool the patient's skin through the window 203 that comes into contact with the skin.

A cartridge board 201a that seals the inside of the cartridge housing 200 is positioned at the upper portion of the cartridge housing 200, and a medium supply protruding tube 210 and a medium discharge protruding tube 220 for circulating the ultrasound transmission medium are provided to protrude on the top of the cartridge board 201a.

An eccentric shaft 500, which is vertically positioned eccentrically from the center of the body of the ultrasonic transducer 300 and connected to a rotating motor 400, is provided on the top of the ultrasonic transducer 300.

The eccentric shaft 500 is vertically positioned at the center of the cartridge housing 200 within the cartridge housing 200, and its lower end is connected to an eccentric position on the top of the ultrasonic transducer 300.

The upper end portion of the eccentric shaft 500 partially protrudes upward through the top of the cartridge board 201a, so it is connected to the rotating motor 400 when the cartridge housing 200 is coupled to the body housing 100.

The rotating motor 400 is connected to the eccentric shaft 500 and transmits rotational force to the eccentric shaft 400, thereby rotating the ultrasonic transducer 300 around the eccentric shaft 500.

The ultrasonic transducer 300 is positioned such that its bottom, which emits ultrasonic waves, is perpendicular to the window 203, so the ultrasonic transducer 300 emits ultrasonic waves in a direction perpendicular to the window 203.

It is exemplified that the rotating motor 400 is positioned inside the body housing 100, and although not shown, it may be mounted on the top of the cartridge board 201a.

When the cartridge housing 200 is coupled to the lower portion of the body housing 100, the portion of the eccentric shaft 500 that protrudes upward through the top of the cartridge board 201a is connected to the rotating motor 400.

The rotating motor 400 is positioned in the body housing 100, and it is connected to the eccentric shaft 500 when the cartridge housing 200 is coupled to the lower portion of the body housing 100, so when the lifespan of the ultrasonic transducer 300 is reached, the cartridge housing unit 200 does not need to be replaced, whereby costs can be reduced.

Though not shown, it should be noted that the rotating motor 400 may be mounted in the cartridge housing 200 and may be replaced together with the cartridge housing 200 when the lifespan of the ultrasonic transducer unit 300 is reached.

The eccentric shaft 500 rotatably protrudes from the top of the cartridge board 201a.

The eccentric shaft 500 is rotatably positioned through the cartridge board 201a and can be achieved using a well-known sealing structure that seals a rotation shaft, so it should be noted that a more detailed description is omitted.

The eccentric shaft 500 includes a connection shaft section 510 protruding upward from the cartridge housing 200 and connected to the rotating motor 400, and a shaft adapter section 410 in which the connection shaft section 510 is inserted to be connected to it is disposed on the shaft of the rotating motor 400.

The shaft adapter section 410 has a shaft insertion portion that is open downward so that the connection shaft section 510 is inserted therein. It is exemplified that the connection shaft section 510 is a shaft having a polygonal cross-section and the shaft insertion portion is a polygonal insertion groove corresponding to the connection shaft section 510.

The connection shaft section 510 is inserted in the polygonal insertion groove of the shaft insertion portion and is rotated by rotational force transmitted from the rotating motor 400.

When the cartridge housing 2000 is coupled, a supply tube connector (not shown) and a discharge tube connector (not shown), which connect the medium supply protruding tube 210 and the medium discharge protruding tube 220 to a medium circulator (not shown) positioned in the control body 1000 controlling operation of the handpiece for ultrasound treatment, are positioned in the body housing 100.

The control body 1000 may be variously modified in well-known ultrasound treatment devices including a controller that controls operation of a handpiece for ultrasound treatment and a medium circulator that circulates an ultrasound transmission medium, so it should be noted that a more detailed description is omitted.

Though not shown, the medium circulator may be variously modified into well-known cooling water circulation structure including a medium storage tank, a medium supply line connecting the medium storage tank and the supply tube connector 120, a medium discharge line connecting the medium storage tank and the discharge tube connector 130, a valve positioned in the medium supply line, and a medium cooler positioned in the medium storage tank. Accordingly, it should be noted that a more detailed description is omitted.

The supply tube connector (not shown) includes a first protruding tube insertion portion in which the medium supply protruding tube 210 is inserted, and the discharge tube connector (not shown) includes a second protruding tube insertion portion in which the medium discharge protruding tube 220 is inserted.

It is exemplified that the medium supply protruding tube 210 is inserted in the first protruding tube insertion portion and connected to the medium supply line when the channel is opened, and the medium discharge protruding tube 220 is inserted in the second protruding tube insertion portion and is connected to the medium discharge line when the channel is opened.

It should be noted that the medium supply protruding tube 210 and the supply tube connector (not shown), and the medium discharge protruding tube 220 and the discharge tube connector (not shown) may be variously modified into well-known tube connection structures including a valve that connects two tubes and is opened when they are connected.

When the cartridge housing 200 is coupled to the body housing 100, the connection shaft section 510 of the eccentric shaft 500 is inserted into the shaft insertion portion of the shaft adapter section 410, whereby the eccentric shaft 500 is connected to the shaft of the rotating motor 400, the medium supply protruding tube 210 is inserted into the first protruding tube insertion portion of the supply pipe connector 120 and is connected to the medium circulator of the control body 1000, and the medium discharge protruding tube 220 is inserted into the second protruding tube insertion portion of the discharge pipe connector 130 and is connected to the medium circulator of the control body 1000.

Further, a pair of first main power supply terminals 230 for supplying power to the ultrasonic transducer 300 is provided on the top of the cartridge board 201a, and though not shown, second main power supply terminals to which the pair of first main power supply terminals 230 is connected and that are connected to the control body 1000 through the handpiece cable assembly 1100 are positioned in the body housing 100.

It is exemplified that the first main power supply terminals 230 are protruding terminals that protrude from the top of the cartridge housing 200, that is, the top of the cartridge board 201a, and the second main power supply terminals are insertion-type terminals in which the first main power supply terminals 230 can be inserted and connected.

When the cartridge housing 200 is coupled to the body housing 100, the pair of first main power supply terminals 230 is inserted into the pair of second main power supply terminals and they are connected to each other, whereby the ultrasonic transducer 300 is connected to the control body 1000 through the handpiece cable assembly 1100, and accordingly, power can be supplied from the control body 1000 and the operation can be controlled.

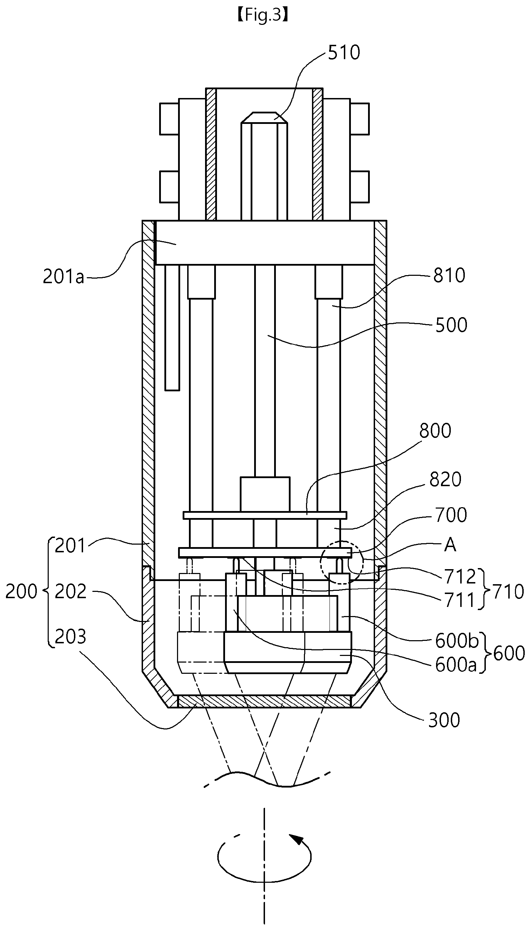

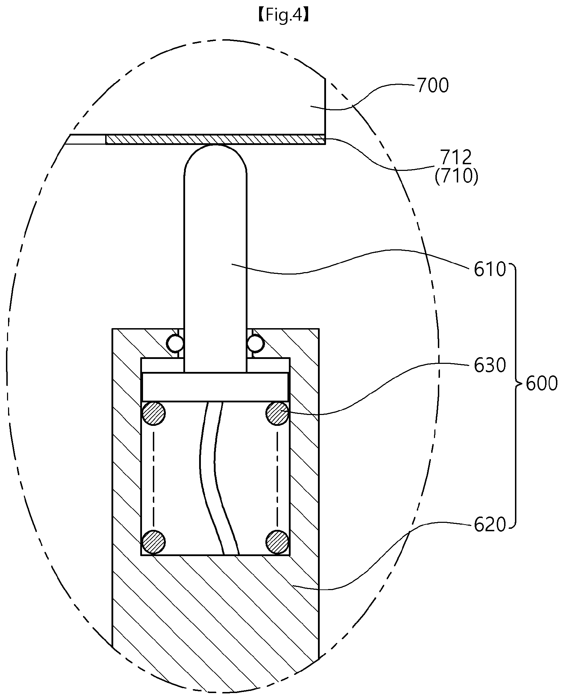

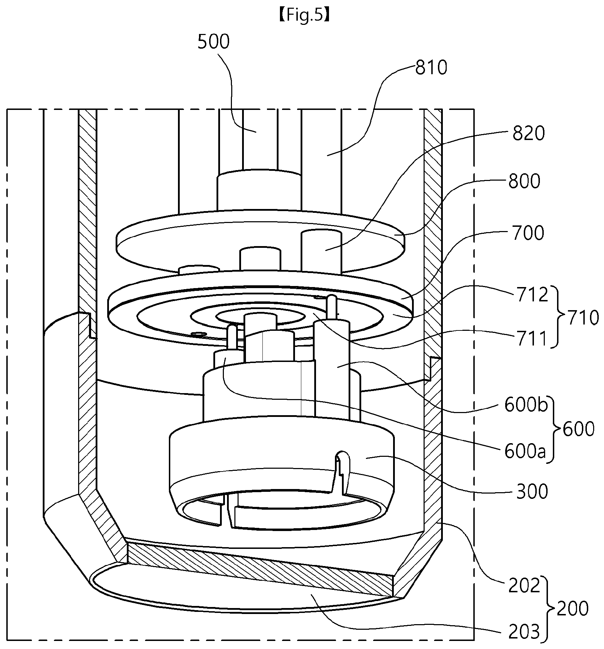

Meanwhile, FIG. 3 is a perspective view showing an embodiment of a therapeutic ultrasound generation device according to the present disclosure, FIG. 4 is an enlarged cross-sectional view of the portion A of FIG. 3, and FIG. 5 is a partial enlarged bottom perspective view showing an embodiment of a therapeutic ultrasound generation device according to the present disclosure.

An embodiment of a therapeutic ultrasound generation device according to the present disclosure is described hereafter in detail with reference to FIG. 2 to FIG. 5.

In an embodiment of a therapeutic ultrasound generation device according to the present disclosure, the ultrasonic transducer 300 is connected to the rotating motor 400 through the eccentric shaft 500 that is offset from the center of body of the ultrasonic transducer 300.

When the ultrasonic transducer 300 is rotated in an eccentric state around the eccentric shaft 500, which is offset, the focus of ultrasonic waves moves in a circular path having a preset radius from the axis center of the rotating motor 400.

The ultrasonic transducer 300 is connected at an eccentric position of the eccentric shaft 500, which is rotated by the rotating motor 400, and rotates on a plane, whereby the focus of the ultrasonic waves generated by the ultrasonic transducer 300 moves in a circular path on the same plane.

The ultrasonic transducer 300 emits ultrasonic waves to the window 203 perpendicularly to the window 203.

The ultrasonic wave emission surface of the ultrasonic transducer 300 is positioned parallel to the window 203 and directs ultrasonic waves perpendicularly to the window 203, and rotates around the eccentric shaft 500 offset from the rotation center of the rotating motor 400, so the focus of the ultrasonic waves moves in a circular path on a plane.

That is, the therapeutic ultrasound generation device according to the present disclosure moves the focus of ultrasonic waves in a circular path on the same plane with a preset radius while maintaining the focus at a uniform depth inside the skin, thereby forming the movement path of the focus as a circle on the plane. Accordingly, the therapeutic ultrasound generation device can uniformly and evenly apply energy within the movement radius of the focus.

Further, the therapeutic ultrasound generation device according to the present disclosure rotates the ultrasonic transducer 300 around the eccentric shaft 500 with the ultrasound emission surface of the ultrasonic transducer 300 positioned parallel to the window section 203, so the movement path of the focus is formed in a circular shape on a plane. Therefore, the output of the ultrasonic waves can be easily controlled, and the movement radius of the focus can be selectively designed to be narrow or wide.

Meanwhile, an embodiment of the therapeutic ultrasound generation device of the present disclosure further includes a power supply protruding terminal unit 600 that protrudes upward from the ultrasonic transducer 300 and supplies power to the ultrasonic transducer 300, and a power supply board 700 that is disposed above and spaced apart from the ultrasonic transducer 300 in the cartridge housing 200 and to which the power supply protruding terminal unit 600 is connected.

A ring-shaped electrode pattern 710 that is connected through a power cable and supplied with power is positioned on the bottom of the power supply board 700.

When the ultrasonic transducer 300 is rotated around the eccentric shaft 500, the power supply protruding terminal unit 600 moves in a circular path with its upper end connected to the ring-shaped electrode pattern 710, whereby poser is stably supplied to the ultrasonic transducer 300.

The ring-shaped electrode pattern 710 is electrically connected to the first main power supply terminals 230, which protrude from the top of the cartridge board 201a, by a power cable through a terminal positioned through the power supply board 700.

Accordingly, when the cartridge housing 200 is coupled to the body housing 100, the pair of first main power supply terminals 230 is inserted into the pair of second main power supply terminals and they are connected to each other, whereby the ring-shaped electrode pattern 710 is connected to the control body 1000 through the handpiece cable assembly 1100 and is supplied with power from the control body 1000 and the power can be stably supplied to the ultrasonic transducer 300 through the power supply protruding terminal unit 600.

The power supply protruding terminal unit 600 maintains in elastic contact with the ring-shaped electrode pattern 710, so when the ultrasonic transducer 300 is rotated around the eccentric shaft 500, the power supply protruding terminal unit 600 moves in a circular path while remaining stably connected to the ring-shaped electrode pattern 710.

As an example, the power supply protruding terminal unit 600 includes a jig pin member 610 that is connected to the ring-shaped electrode pattern 710 and is electrically connected to the ultrasonic transducer 300 through a wire, a pin insertion member 620 that is positioned on the top of the ultrasonic transducer 300 and in which the jig pin member 610 is positioned to be movable up and down, and a pin support spring member 630 that is positioned in the pin insertion member 620 and elastically supports the jig pin member 610.

The jig pin member 610 is elastically supported by the pin support spring member 630, with the upper end in contact with the ring-shaped electrode pattern 710.

When the ultrasonic transducer 300 is eccentrically rotated around the eccentric shaft 500 by the rotating motor 400, the jig pin member 610 elastically supported by the pin support spring member 630 with the upper end in contact with the ring-shaped electrode pattern 710 moves in a circular path along the ring-shaped electrode pattern 710, whereby power can be stably supplied to the ultrasonic transducer 300.

In more detail, the power supply protruding terminal unit 600 includes a cathode protruding terminal unit 600a and an anode protruding terminal unit 600b, which are positioned at different distances from the eccentric shaft 500, and the ring-shaped electrode pattern 710 includes a first electrode pattern 711 with a radius corresponding to the rotation radius of the cathode protruding terminal unit 600a and a second electrode pattern 712 with a radius corresponding to the rotation radius of the anode protruding terminal unit 600b.

The cathode protruding terminal unit 600a and the anode protruding terminal unit 600b are in contact with the first electrode pattern 711 and the second electrode pattern 712, which have different radii, respectively, that is, are positioned with the jig pin members 610 in elastic contact with them, and moves in a circular path while remaining in contact with the ring-shaped first and second electrode patterns 711 and 712, respectively, whereby power can be stably supplied to the ultrasonic transducer section 300.

Further, since the ultrasonic transmission medium is an insulating liquid, even if the power supply protruding terminal unit 600 is exposed within the cartridge housing section 200, no short circuit occurs, and power can be stably supplied into the ultrasonic transducer 300 with the cathode protruding terminal unit 600a and the anode protruding terminal unit 600b in contact with the first electrode pattern 711 and second electrode pattern 712 that are the two exposed ring-shaped electrode patterns.

Further, an embodiment of the therapeutic ultrasound generation device according to the present disclosure may further include: a board position-fixing board 800 that is positioned between the cartridge board 201a and the power supply board 700 and supports the position of the power supply board 700; a plurality of first board fixing portions 810 both ends of which are fixed to the cartridge board 201a and the board position-fixing board 800 and that supports the position of the board position-fixing board 800; and a plurality of second board fixing portions 820 both ends of which are fixed to the board position-fixing board 800 and the power supply board 700 and that supports the position of the power supply board 700.

The eccentric shaft 500 is connected at an eccentric position on the top of the ultrasonic transducer 300 through the board position-fixing board 800 and the power supply board 700.

The board position-fixing board 800 is fixed in position by being connected to the top of the cartridge housing 200, that is, to the cartridge board 201a, by the plurality of first board fixing portions 810, and the power supply board 700 is stably fixed in position by being connected to the board position-fixing board 800 by the plurality of second board fixing portions 820.

The plurality of first board fixing portions 810 and the plurality of second board fixing portions 820 are formed in a hollow tubular shape, and power cables connecting the ring-shaped electrode pattern 710 and the first main power supply terminal 230 can be disposed inside them.

The power cable connecting the ring-shaped electrode pattern 710 and the first main power supply terminal 230 may not be exposed to the outside by being disposed through the insides of the plurality of first board fixing portion 810 and the plurality of second board fixing portions 820.

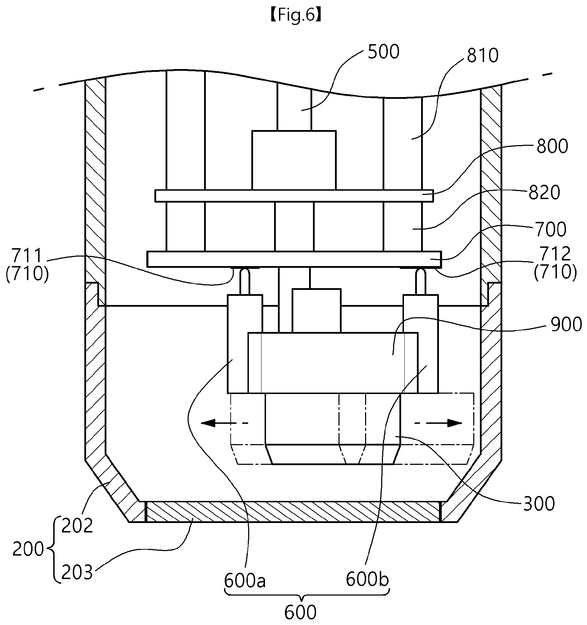

FIG. 6 is a partial enlarged view showing another embodiment of a therapeutic ultrasound generation device according to the present disclosure. Referring to FIG. 6, another embodiment of the therapeutic ultrasound generation device according to the present disclosure further includes an ultrasound transducer displacement unit 900 under which the ultrasound transducer 300 is movably positioned and to the upper portion of which the eccentric shaft 500 is connected.

The ultrasound transducer displacement unit 900 linearly moves the ultrasound transducer 300 in the circumferential direction from the rotation center of the rotating motor 400 on its bottom, thereby being able to adjust the rotation radius of the ultrasound transducer 300 with respect to the rotation center of the rotating motor 400.

The power supply protruding terminal unit 600 is mounted on the ultrasound transducer displacement unit 900, the ultrasound transducer displacement unit 900 is connected to the rotating motor 400 through the eccentric shaft 500, and the ultrasound transducer 300 is positioned under the ultrasound transducer displacement unit 900 so that it can horizontally linearly move.

The ultrasound transducer 300 is linearly moved by the ultrasound transducer displacement unit 900, whereby the rotation radius by the eccentric shaft 500 can be adjusted.

That is, another embodiment of the therapeutic ultrasound generation device according to the present disclosure allows the ultrasound transducer 300 to be moved bidirectionally in a straight direction on a plane passing through the eccentric shaft 500, thereby allowing the movement radius of the focus, which is formed in a circular shape on the plane, to be adjusted in various ways.

Another embodiment of the therapeutic ultrasound generation device according to the present disclosure can variably adjust the movement radius of the focus, which is formed in a circular shape on a plane, depending on the size of the treatment area or the treatment effect.

The present disclosure applies energy uniformly and evenly to a treatment area by moving the focus of ultrasonic waves at a uniform depth within the skin in a planar manner, and applies energy uniformly and evenly within a radius by forming the focus of the ultrasonic waves at a uniform depth within the skin in a circular shape with a predetermined radius, thereby being able to improve treatment performance.

The present disclosure can further improve the treatment effect by moving the focus of ultrasonic waves in a circular path on a plane by rotating the ultrasonic transducer 300 at an offset position on the plane to make it easy to adjust the output of ultrasonic waves, and can increase the versatility of the treatment by expanding the range of the movement radius of the focus.

Further, the present disclosure can minimize the load on the motor and reduce manufacturing costs by simplifying the structure for moving the focus of the ultrasonic waves generated by the ultrasonic transducer 300 in a circular path on the same plane.

The present disclosure is not limited to the embodiments described above and may be modified in various ways without departing from the scope of the present disclosure and the modifications should be construed as being included in the present disclosure.

## Claims

1. A therapeutic ultrasound generation device comprising:
a cartridge housing having, on a bottom thereof, a window configured to come into contact with the skin, through which ultrasonic pass;
an ultrasound transducer positioned to rotate on a plane in the cartridge housing and configured to generate ultrasonic waves;
a rotating motor configured to rotate the ultrasound transducer; and
an eccentric shaft eccentrically connected to a center of body of the ultrasound transducer on a top of the ultrasound transducer, and configured to rotate by rotational force transmitted from the rotating motor.

2. The therapeutic ultrasound generation device of claim 1, wherein the ultrasound transducer is rotated around the eccentric shaft, whereby a focus of ultrasonic waves generated by the ultrasound transducer moves in a circular path on a plane.

3. The therapeutic ultrasound generation device of claim 1, further comprising:
a power supply protruding terminal unit protruding upward from the ultrasound transducer and configured to supply power to the ultrasound transducer; and
a power supply board disposed above and spaced apart from the ultrasonic transducer in the cartridge housing, to which the power supply protruding terminal unit is connected.

4. The therapeutic ultrasound generation device of claim 3, wherein the cartridge housing has a sealed internal structure and is filled with an ultrasound transmission medium, and
the ultrasound transmission medium is an insulating liquid.

5. The therapeutic ultrasound generation device of claim 3, wherein a ring-shaped electrode pattern connected through a power cable to receive power is positioned on a bottom of the power supply board, and
the power supply protruding terminal unit moves in a circular path along the ring-shaped electrode pattern in elastic contact with the ring-shaped electrode pattern.

6. The therapeutic ultrasound generation device of claim 5, wherein the power supply protruding terminal unit comprises:
a jig pin member connected to the ring-shaped electrode pattern and electrically connected to the ultrasound transducer through a wire;
a pin insertion member positioned on the top of the ultrasonic transducer, in which the jig pin member is positioned to be movable up and down; and
a pin support spring member positioned in the pin insertion member and configured to elastically support the jig pin member.

7. The therapeutic ultrasound generation device of claim 5, further comprising:
a board position-fixing board positioned between a top of the cartridge housing and the power supply board and configured to support the position of the power supply board;
a plurality of first board fixing portions having both ends fixed to the top of the cartridge housing and the board position-fixing board and configured to support the position of the board position-fixing board; and
a plurality of second board fixing portions having both ends fixed to the board position-fixing board and the power supply board and configured to support the position of the power supply board.

8. The therapeutic ultrasound generation device of claim 7, wherein the plurality of first board fixing portions and the plurality of second board fixing portions are formed in a hollow tubular shape, and power cables connecting the ring-shaped electrode pattern and a first main power supply terminal protruding upward from the cartridge housing are disposed inside the first and second board fixing portions.

9. The therapeutic ultrasound generation device of claim 2, further comprising an ultrasound transducer displacement unit under which the ultrasound transducer is movably positioned and to an upper portion of which the eccentric shaft is connected, thereby being able to adjust a movement radius of the focus on a plane.

10. A therapeutic ultrasound generation device comprising:
a body housing connected to a control body through a handpiece cable assembly; and
a therapeutic ultrasound generation device detachably coupled to the body housing,
wherein the therapeutic ultrasound generation device is the therapeutic ultrasound generation device of any one of claims 1 to 9.
